# EUROPÄISCHE PATENTSCHRIFT

(11) **EP 3 122 691 B1**
(45) Veröffentlichungstag und Bekanntmachung des Hinweises auf die Patenterteilung: **29.06.2022**
(21) Anmeldenummer: 15715195.2
(22) Anmeldetag: 27.03.2015
(51) Int. Cl.: C03C 3/097, C03C 4/00, C03C 10/00

(54) **VERFAHREN ZUR HERSTELLUNG EINES LITHIUMSILIKAT-GLASROHLINGS UND EINES LITHIUMSILIKAT-GLASKERAMIKROHLINGS**
METHOD FOR PRODUCING A LITHIUM SILICATE GLASS PREFORM AND A LITHIUM SILICATE GLASS CERAMIC PREFORM
PROCÉDÉ DE FABRICATION D'UNE ÉBAUCHE EN VERRE DE SILICATE DE LITHIUM ET D'UNE ÉBAUCHE EN VITROCÉRAMIQUE DE SILICATE DE LITHIUM

(30) Priorität: 28.03.2014 DE 102014104401
(43) Veröffentlichungstag der Anmeldung: 01.02.2017
(73) Patentinhaber: Dentsply Sirona Inc., York, PA 17401 (US)
(72) Erfinder: VOLLMANN, Markus, 63571 Gelnhausen (DE); SCHUSSER, Udo, 63755 Alzenau (DE)
(74) Vertreter: Pichova, Vanda
(86) Internationale Anmeldenummer: PCT/EP2015/056675
(87) Internationale Veröffentlichungsnummer: WO 2015/144866

(56) Entgegenhaltungen:
- EP-A1- 2 662 342
- WO-A1-2012/175450
- DE-A1-102009 060 274
- GB-A- 2 284 655
- US-A1- 2003 022 782
- US-B1- 6 184 162

## Beschreibung

Die Erfindung bezieht sich auf ein Verfahren zur Herstellung eines Lithiumsilikat-Glasrohlings mit einer Zusammensetzung mit zumindest 8 Gew.-%, vorzugsweise 9 bis 20 Gew.-%, eines Stabilisators aus der Gruppe ZrO₂, HfO₂ oder Mischungen dieser. Auch nimmt die Erfindung Bezug auf ein Verfahren zur Herstellung eines Lithiumsilikat-Glaskeramikrohlings und der Verwendung des Lithiumsilikat-Glaskeramikrohlings. Die Erfindung nimmt auch Bezug auf die Verwendung des Lithiumsilikat-Glaskeramikrohlings sowie auf ein Dentalprodukt.

Rohlinge aus Lithiumsilikat-Glaskeramik haben sich im Bereich der Zahntechnik aufgrund ihrer Festigkeit und Biokompatibilität bewährt. Dabei zeigt sich der Vorteil, dass dann, wenn ein Lithiumsilikat-Rohling Lithiummetasilikat als Hauptkristallphase enthält, eine problemlose maschinelle Bearbeitung möglich ist, ohne dass ein hoher Werkzeugverschleiß auftritt. Erfolgt sodann eine Wärmebehandlung, in der das Produkt in eine Lithiumdisilikat-Glaskeramik umgewandelt wird, ergibt sich eine hohe Festigkeit. Auch sind gute optische Eigenschaften und eine gute chemische Stabilität gegeben. Entsprechende Verfahren sind der DE 197 50 794 A1 oder der DE 103 36 913 B4 zu entnehmen.

Es hat sich gezeigt, dass die Festigkeit erhöht und eine gute Transluzenz erzielbar ist, wenn den Ausgangsrohstoffen in Form von Lithiumkarbonat, Quarz, Aluminiumoxid etc., also üblichen Anfangskomponenten, zumindest ein Stabilisator aus der Gruppe Zirkonoxid, Hafniumoxid oder Mischungen dieser, insbesondere Zirkonoxid, beigegeben wird. Der Gewichtsanteil des Stabilisators in der Ausgangsmischung kann dabei bis zu 20 % betragen. Insoweit wird beispielhaft auf die DE 10 2009 060 274 A1 oder die WO 2012/175450 A1, die WO 2012/175615 A1, die WO 2013/053865 A2 oder die EP 2 662 342 A1 verwiesen.

In der Praxis zeigen sich jedoch dahingehend Probleme, dass nach der finalen Kristallisation, also insbesondere dann, wenn in dem Lithiumsilikat-Glas als Hauptkristallisationsphase Lithiumdisilikat vorliegt, die Stabilisatoren, insbesondere Zirkonoxid, rekristallisieren, gleichwenn dieses zuvor in der Glasphase des Lithiumsilikats vollständig gelöst war.

Der vorliegenden Erfindung liegt u. a. die Aufgabe zu Grunde, ein Verfahren zur Herstellung eines Lithiumsilikat-Glasrohlings zur Verfügung zu stellen, das sicherstellt, dass der Stabilisator auch beim anschließenden Wärmebehandeln, insbesondere dann, wenn Lithiumdisilikat als Hauptkristallphase vorliegen soll, nicht rekristallisiert.

Nach einem weiteren Aspekt soll sichergestellt sein, dass die abzufüllende Schmelze im industriellen und reproduzierbaren Maßstab zu insbesondere dentalen Produkten wie Presspellets oder mittels CAD/CAM-Verfahren zu bearbeitenden Blöcken geformt werden kann. Es soll verhindert werden, dass beim Befüllen der Formen ein sogenanntes "Hochschwappen" der Schmelze im Forminneren erfolgt, so dass die gewünschte glatte, horizontal verlaufende Oberfläche des erstarrten Formkörpers nicht erzielbar wäre.

Zur Lösung eines oder mehrerer Aspekte sieht die Erfindung vor ein Verfahren zur Herstellung eines Lithiumsilikat-Glasrohlings mit zumindest 8 Gew.-%, vorzugsweise 9 bis 20 Gew.-%, eines Stabilisators aus der Gruppe ZrO₂, HfO₂ oder Mischungen dieser, das die Verfahrensschritte umfasst:
- Mischen von den Stabilisator in Form von Pulver enthaltenden Rohstoffen, wobei das Pulver eine Korngröße d₅₀ = x mit 0,3 µm ≤ x ≤ 1,5 µm aufweist,
- Aufschmelzen der Rohstoffe bei einer Temperatur T_{AU} mit 1450 °C ≤ T_{AU} ≤ 1600 °C in einem Behälter und Halten der Schmelze in dem Behälter über eine Zeit t_{H} mit t_{H} ≥ 1 h,
- Abfüllen der homogenisierten Schmelze in Behältnisse, wobei die Abflusstemperatur T_{AB} aus dem Behälter beträgt T_{AU} - Y °C = T_{AB} mit 150 °C ≤ Y ≤ 350 °C, wobei das Befüllen der Behältnisse und

Formgeben der Schmelze in diesen mit einer Abkühlrate A mit 5 K/sec ≤ A ≤ 100 K/sec bis zu einer Temperatur T_{M} mit 600 °C ≤ T_{M} , insbesondere 600 °C ≤ T_{M} ≤ 650 °C, erfolgt.

Überraschenderweise hat sich gezeigt, dass dann, wenn das Stabilisatorpulver, insbesondere Zirkonoxidpulver, eine Korngröße d₅₀ zwischen 0,3 µm und 1,5 µm aufweist, das Zirkonoxid gut in Lösung geht und dort verbleibt, also in der amorphen Phase, d. h. dem Glas vorliegt, und zwar auch dann, wenn die Schmelze in die Form gefüllt, abgekühlt und sodann ein oder mehreren Wärmebehandlungen zur Bildung von zumindest Lithiumdisilikat-Kristallen ausgesetzt wird. Gleichzeitig ist der Vorteil gegeben, dass Probleme mit einer Agglomeration nicht auftreten.

Die Angaben d₅₀, d₁₀, d₉₀ bedeuten, dass 50 % bzw. 10 % bzw. 90 % der Partikel eine Korngröße aufweisen, die kleiner als der angegebene Wert der Korngröße ist.

Um das "Hochschwappen" zu vermeiden, hat sich als vorteilhaft erwiesen, wenn die Glasschmelze mit einer Abflusstemperatur T_{AB} 1200 °C ≤ T_{AB} ≤ 1350 °C, vorzugsweise 1250 °C ≤ T_{AB} ≤ 1300 °C aus dem Behälter fließt, wobei bis zum Abfüllen der Formen eine Abkühlung bis insbesondere im Bereich von 1150 °C möglich ist, ohne dass Nachteile bei der Endform der Lithiumsilikat-Glasrohlinge nach Abkühlen der Schmelze in der Form feststellbar sind.

Durch das Abkühlen der Schmelze im Vergleich zu der Temperatur, bei der die Ausgangsstoffe geschmolzen und durch Thermik homogenisiert werden, ohne dass es mechanischer Hilfsmittel wie Rührer bedarf, ergibt sich der Vorteil, dass die Schmelze eine Viskosität aufweist, die das reproduzierbare Befüllen der Formen bei gleichzeitiger Erzielung einer horizontal verlaufenden Oberfläche ermöglicht. Das Verhindern der Rekristallisation wird möglicherweise auch dadurch unterstützt, dass die Schmelze in der Form mit einer Abkühlrate im Bereich zwischen 5 K/sec und 100 K/sec abkühlt, wodurch verhindert wird, dass sich Keime für das Stabilisatormaterial bilden. Die Abkühlrate gilt zumindest bis zu einer Temperatur T_{M} ≥ 600 °C, insbesondere 600 °C ≤ T_{M} ≤ 650 °C.

Anschließend kann ein Abkühlen auf Raumtemperatur in üblicher Art und Weise erfolgen.

Wichtig ist auch das Homogenisieren der Schmelze, wobei sich als vorteilhaft erwiesen hat, wenn die Schmelze bei der Temperatur T_{AU}, die zwischen 1500 °C und der Warmfestigkeit des verwendeten Tiegelmaterials, wie Platinlegierung, liegt, über einen Zeitraum von zumindest 1 Stunde, insbesondere über einen Zeitraum zwischen 2 und 7 Stunden gehalten wird. Ein mehrfaches Aufschmelzen ist nicht erforderlich.

Die Erfindung zeichnet sich folglich auch dadurch aus, dass nach Schmelzen der Rohstoffe in dem Behälter und Homogenisieren in diesem, insbesondere durch Thermik, die Schmelze unmittelbar in die Behältnisse abgefüllt wird.

Folglich zeichnet sich die Erfindung und in Abweichung vom Stand der Technik bevorzugterweise dadurch aus, dass die Herstellung einer Fritte und das Wiederaufschmelzen dieser nicht erforderlich sind, ohne dass sich Nachteile hinsichtlich des Homogenisierens ergeben. Somit ergibt sich im Vergleich zum Stand der Technik grundsätzlich eine kürzere Verfahrensdauer bzw. eine kostengünstigere Herstellung des Rohlings.

Die Erfindung wird selbstverständlich nicht verlassen, wenn eine Fritte hergestellt wird, die wieder aufgeschmolzen wird.

Auch besteht die Möglichkeit, dass die Schmelze in dem Behälter, die durch Thermik homogenisiert wird, während des Homogenisierens abgekühlt wird. So kann die Schmelze z. B. zunächst über eine Zeitdauer von 2 bis 6 Stunden auf einer Temperatur T₁ mit 1450 °C ≤ T₁ ≤ 1550 °C und sodann über eine Zeit t₂ auf einer Temperatur 1200 °C ≤ t₂ ≤ 1300 °C gehalten werden, um anschließend zum Befüllen der Formen abgegossen zu werden bzw. abzufließen.

Insbesondere zeichnet sich die Erfindung dadurch aus, dass das verwendete Pulver des Weiteren eine Korngröße d₁₀ = 0,5 • x und/oder d₉₀ = 1,5 • x, insbesondere d₁₀ = 0,5 • x und d₉₀ = 1,5 • x, mit 0,3 µm ≤ x ≤ 1,5 µm aufweist.

Durch die diesbezüglichen Nebenbedingungen ist sichergestellt, dass der Anteil kleiner Körner so gering ist, dass ein Agglomerieren nicht erfolgt. Durch die Begrenzung der Anzahl der großen Körner ist gleichfalls ein Lösen des Stabilisators im erforderlichen Umfang sichergestellt.

Insbesondere ist vorgesehen, dass der Stabilisator mehr als 90 Gew.-% ZrO₂, insbesondere mehr als 95 Gew.-% ZrO₂, bevorzugterweise mehr als 97, 5 Gew.-% ZrO₂ enthält.

Bevorzugterweise weist die Zusammensetzung des Rohlings in Gew.-% folgende Bestandteile auf:

| | |
|---|---|
| SiO2 | 46,0 - 72,0 |
| Li2O | 10,0 - 25,0 |
| ZrO₂ | 8,0 - 20,0 |
| Al2O3 | 0,1 - 8,0 |
| K2O | 0,1 - 5,0 |
| CeO2 | 0,0 - 4,0 |
| B2O3 | 0,0 - 4,0 |
| Na2O | 0,0 - 4,0 |
| Tb4O7 | 0,0 - 2,5 |
| zumindest einen Keimbildner 1,0 - 10,0 | |
| wie P2O5 | |
| sowie 0,0 bis 4,0 von zumindest einem Additiv, | |

wobei die Gesamtsumme 100 Gew.-% ausmacht.

Das Additiv kann zumindest ein Oxid aus Gruppe sein: BaO, CaO, MgO, MnO, Er₂O₃, Pr₆O₁₁, Sm₂O₃, TiO₂, V₂O₅, Y₂O₃

Des Weiteren ist die Erfindung gekennzeichnet durch ein Verfahren zur Herstellung eines Lithiumsilikat-Glaskeramikrohlings unter Verwendung des Lithiumsilikat-Glasrohlings hergestellt nach einem oder mehreren der zuvor erläuterten Verfahrensschritte, wobei die in den Behältnissen abgefüllte und in diesen abgekühlte Schmelze zumindest einer ersten Wärmebehandlung W1 bei einer Temperatur T_{W1} über einen Zeitraum t_{W1} unterzogen wird, wobei 620 °C ≤ T_{W1}≤ 800 °C, insbesondere 650 °C ≤ T_{W1} ≤ 750 °C, und/oder 1 min ≤ t_{W1} ≤ 200 min, vorzugsweise 10 min ≤ t_{W1} ≤ 60 min, ist.

Durch diesen Schritt entstehen Keimbildner und Lithiummetasilikat-Kristalle.

Ein entsprechender Lithiumsilikat-Glaskeramikrohling ist problemlos bearbeitbar, wobei der Werkzeugverschleiß minimal ist. Auch kann ein entsprechender Rohling in eine gewünschte Geometrie verpresst werden.

Insbesondere ist zur Erzielung einer finalen Kristallisation, insbesondere zur Bildung von Lithiumdisilikat-Kristallen bzw. Umwandeln der Metasilikat-Kristalle in Disilikat-Kristalle vorgesehen, dass der Lithiumsilikat-Glaskeramikrohling nach der ersten Wärmebehandlung W1 einer zweiten Wärmebehandlung W2 bei einer Temperatur T_{W2} über eine Zeit t_{W2} unterzogen wird, wobei 800 °C < T_{W2} ≤ 1040 °C, vorzugsweise 800 °C ≤ T_{W2} ≤ 900 °C, und/oder 5 min ≤ t_{W2} ≤ 200 min, vorzugsweise 5 min ≤ t_{W2} ≤ 30 min, ist.

Vorzugsweise werden bei den zu einer Keimbildung und Vorkristallisation bzw. finalen Kristallisation führenden Wärmebehandlungsschritten folgende Temperaturwerte und Aufheizraten gewählt. Bezüglich der ersten Wärmebehandlung ist insbesondere vorgesehen, dass diese zweistufig erfolgt, wobei eine erste Haltestufe im Bereich zwischen 640 °C und 680 °C und eine zweite Haltestufe zwischen 720 °C und 780 °C liegt. In jeder Stufe wird der aufgeheizte Formling über einen Zeitraum gehalten, wobei in der ersten Stufe ein Zeitraum bevorzugterweise zwischen 35 und 45 Minuten und in der zweiten Stufe zwischen 15 und 25 Minuten liegt.

Entsprechende Lithiumsilikat-Glaskeramikrohlinge weisen eine hohe Transluzenz und chemische Beständigkeit auf. Sie zeichnen sich auch durch ihre Festigkeit aus. Eine Ausscheidung von Stabilisatormaterial, insbesondere Zirkonoxid ist nicht feststellbar. Daher sind entsprechende Lithiumsilikat-Glaskeramikrohlinge insbesondere als Dentalmaterialien oder Komponenten solcher geeignet, wobei geformte Dentalprodukte in Form von z. B. Inlays, Onlays, Brücken, Verblendungen, Veneere, Facetten, Kronen, Teilkronen, Abutments Verwendung finden können.

Insbesondere zeigen entsprechende Lithiumsilikat-Glaskeramikrohlinge eine extrem gute Bearbeitung mittels CAD/CAM, wobei nach der weiteren Wärmebehandlung ein hochtransparentes und hochfestes Produkt zur Verfügung steht, das eine hohe chemische Beständigkeit aufweist.

Weitere Einzelheiten, Vorteile und Merkmale der Erfindung ergeben sich nicht nur aus den Ansprüchen und den diesen zu entnehmenden Merkmalen - für sich und/oder in Kombination -, sondern auch aus den nachstehenden Beispielen.

Es wurden Rohstoffe, wie Lithiumcarbonat, Quarz, Aluminiumoxid, Zirkondioxid im industriellen Maßstab mittels eines Taumelmischers gemischt, bis ein visuell einheitliches Gemenge vorlag. Dabei wurden insgesamt 5 Gemenge hergestellt, die sich darin unterschieden, dass das Zirkondioxid in der Korngröße voneinander abweichende Werte aufwies.

Die Zusammensetzung der Probenkörper für die durchgeführten Versuche in Gew.-% war folgende:

| | |
|---|---|
| SiO2 | 58 - 60 |
| Li2O | 13,5 - 20,5 |
| ZrO₂ | 9,0 - 12,5 |
| P2O5 | 3,0 - 7,5 |
| Al2O3 | 0,5 - 6,0 |
| K2O | 0,5 - 3,5 |
| CeO2 | 0,5 - 2,5 |
| B2O3 | 0 - 3 |
| Na2O | 0 - 3 |
| Tb4O7 | 0 - 1,5. |

### Beispiel 1:

Es wurde ein Zirkonoxid-Pulver mit einer Korngröße d₅₀ = etwa 15 µm, d₁₀ = 10,8 µm, d₉₀ = 34,9 µm benutzt.

Das Gemenge wurde in einem hochtemperaturfesten Tiegel aus Platinlegierung über eine Zeit von 2,25 Stunden bei 1450 °C aufgeschmolzen. Die Schmelze wurde sodann abgekühlt, zunächst über eine halbe Stunde auf 1450 °C und sodann über eine halbe Stunde auf 1250 °C gehalten. Anschließend wurde die Schmelze in Formen gegossen, die für Presspellets oder Blöcke eines Volumens von 1 cm³ bis 2 cm³ geeignet sind, um eine maschinelle Bearbeitung vorzunehmen. Die Abkühlrate betrug 70 K/sec bis 600 °C. sodann erfolgte eine Abkühlung auf Raumtemperatur. Es zeigte sich ein amorphes und somit transparentes Glas. Sodann wurden die Formkörper einem Kristallisationsbrand unterzogen, wobei in einer ersten Wärmebehandlung die Formkörper über 60 Minuten auf 660 °C und sodann in einer zweiten Wärmebehandlung über 8 Minuten auf 850 °C gehalten worden sind. Anschließend erfolgte eine Abkühlung auf Raumtemperatur. Bei einer Überprüfung der Glaskeramik wurden einzelne Zirkonoxid-Ausscheidungen festgestellt, die die Glaskeramik opak werden ließ.

### Beispiel 2:

Es wurde ein Zirkonoxid-Pulver mit einer Korngröße d₅₀ = etwa 0,7 µm, d₁₀ = 0,2 µm, d₉₀ = 2,2 µm benutzt.

Das Gemenge wurde in einem hochtemperaturfesten Tiegel aus Platinlegierung bei einer Temperatur T = 1500 °C aufgeschmolzen und bei dieser Temperatur über 6 Stunden gehalten. Anschließend wurde die Schmelze in Formen gegossen, die für Presspellets oder Blöcke eines Volumens von 1 cm³ bis 2 cm³ geeignet sind, um eine maschinelle Bearbeitung vorzunehmen. Die Abkühlrate betrug 70 K/sec bis 600° C. Sodann erfolgte eine Abkühlung auf Raumtemperatur. Es zeigte sich ein amorphes und somit transparentes Glas. Sodann wurden die Formkörper einem Kristallisationsbrand unterzogen. Zur Keimbildung bzw. Vorkristallisation wurde das Glas zunächst von Raumtemperatur mit einer Aufheizrate von 2 K/min auf 660 °C erwärmt und bei dieser Temperatur 40 Minuten gehalten. Anschließend erfolgte eine weitere Erwärmung auf 750 °C mit einer Aufheizrate von 10 K/min. Die Haltezeit betrug 20 Minuten. Anschließend erfolge die finale Kristallisation bei einer Temperatur von 850 °C über 8 Minuten. Sodann erfolgte eine Abkühlung auf Raumtemperatur. Bei einer Überprüfung der Glaskeramik wurden keine Zirkonoxid-Ausscheidungen festgestellt.

### Beispiel 3:

Es wurde ein Zirkonoxid-Pulver mit einer Korngröße d₅₀ = etwa 0,7 µm, d₁₀ = 0,2 µm, d₉₀ = 2,2 µm benutzt.

Das Gemenge wurde in einem hochtemperaturfesten Tiegel aus Platinlegierung über eine Zeit von 6 Stunden bei 1500 °C aufgeschmolzen. Die Schmelze wurde sodann abgekühlt und über eine halbe Stunde auf 1250 °C gehalten. Anschließend wurde die Schmelze in Formen gegossen, die für Presspellets oder Blöcke eines Volumens von 1 cm³ bis 2 cm³ geeignet sind, um eine maschinelle Bearbeitung vorzunehmen. Die Abkühlrate betrug 70 K/sec bis 600 °C. Sodann wurde auf Raumtemperatur abgekühlt. Es zeigte sich ein amorphes und somit transparentes Glas. Sodann wurden die Formkörper einem Kristallisationsbrand unterzogen. Zur Keimbildung bzw. Vorkristallisation wurde das Glas zunächst von Raumtemperatur mit einer Aufheizrate von 2 K/min auf 660 °C erwärmt und bei dieser Temperatur 40 Minuten gehalten. Anschließend erfolgte eine weitere Erwärmung auf 750 °C mit einer Aufheizrate von 10 K/min. Die Haltezeit betrug 20 Minuten. Anschließend erfolgte die finale Kristallisation bei einer Temperatur von 850 °C über 8 Minuten. Sodann erfolgte eine Abkühlung auf Raumtemperatur. Bei einer Überprüfung der Glaskeramik wurden keine Zirkonoxid-Ausscheidungen festgestellt.

### Beispiel 4:

Es wurde ein Zirkonoxid-Pulver mit einer Korngröße dso = etwa 5 µm, d₁₀ = 0,3 µm, d₉₀ = 5,8 µm benutzt.

Das Gemenge wurde in einem hochtemperaturfesten Tiegel aus Platinlegierung über eine Zeit von 4 Stunden bei 1500 °C aufgeschmolzen. Die Schmelze wurde sodann über 1 Stunde auf 1450 °C gehalten. Anschließend wurde die Schmelze in Formen gegossen, die für Presspellets oder Blöcke eines Volumens von 1 cm³ bis 2 cm³ geeignet sind, um eine maschinelle Bearbeitung vorzunehmen. Die Abkühlrate bis 600 °C betrug 70 K/sec. Anschließend wurde auf Raumtemperatur abgekühlt. Es zeigte sich ein amorphes und somit transparentes Glas. Sodann wurden die Formkörper einem Kristallisationsbrand unterzogen, wobei in einer ersten Wärmebehandlung die Formkörper über 60 Minuten auf 620 °C (Vorkristallisation) und sodann in einer zweiten Wärmebehandlung über 8 Minuten auf 850 °C (finale Kristallisation) gehalten worden sind. Anschließend erfolgte eine Abkühlung auf Raumtemperatur. Bei einer Überprüfung der Glaskeramik wurden zahlreiche kleine Zirkonoxid-Ausscheidungen festgestellt, die die Glaskeramik opak werden ließ.

Aus den zuvor wiedergegebenen Beispielen ergibt sich, dass bei Verwendung von Zirkonoxid-Pulver mit einer Korngröße d₅₀ = 0,7 µm, einer Aufschmelztemperatur von 1500 °C und einer gegenüber dieser niedrigeren Abfließtemperatur Glaskeramikkörper herstellbar sind, die Zirkonoxid-Ausscheidungen nicht zeigen. Die Glaskeramikkörper wiesen eine hohe Transluzenz auf. Chemische und mechanische Überprüfungen zeigen eine hohe Beständigkeit und Festigkeit.

## Patentansprüche

1. Verfahren zur Herstellung eines Lithiumsilikat-Glasrohlings mit einer Zusammensetzung mit zumindest 8 Gew.-%, vorzugsweise 9 bis 20 Gew.-%, eines Stabilisators aus der Gruppe ZrO₂, HfO₂ oder Mischungen dieser, umfassend die Verfahrensschritte
- Mischen von den Stabilisator in Pulverform enthaltenden Rohstoffen, wobei das Pulver eine Korngröße d₅₀ = x mit 0,3 µm ≤ x ≤ 1,5 µm aufweist,
- Aufschmelzen der Rohstoffe bei einer Temperatur T_{AU} mit 1450 °C ≤ T_{AU} ≤ 1600 °C in einem Behälter und Halten der Schmelze in dem Behälter über eine Zeit t_{H} mit t_{H} ≥ 1 h,
- Abfüllen der homogenisierten Schmelze in Behältnisse, wobei die Abflusstemperatur T_{AB} aus dem Behälter beträgt T_{AU} - Y °C = T_{AB} mit 150 °C ≤ Y ≤ 350 °C, wobei das Befüllen der Behältnisse und Formgeben der Schmelze in diesen mit einer Abkühlrate A mit 5 K/sec ≤ A ≤ 100 K/sec bis zu einer Temperatur T_{M} mit 600 °C ≤ T_{M} , insbesondere 600 °C < T_{M} ≤ 650 °C, erfolgt.

2. Verfahren nach Anspruch 1,
**dadurch gekennzeichnet,**
**dass** das verwendete Pulver des Weiteren eine Korngröße d₁₀ = 0,5 • x und/oder d₉₀ = 1,5 • x, insbesondere d₁₀ = 0,5 • x und d₉₀ = 1,5 • x, mit 0,3 µm ≤ x ≤ 1,5 µm aufweist.

3. Verfahren nach Anspruch 1 oder Anspruch 2,
**dadurch gekennzeichnet,**
**dass** die Schmelze beim Befüllen der Behältnisse eine Temperatur T_{B} mit 1150 °C ≤ T_{B} < T_{AB} aufweist.

4. Verfahren nach Anspruch 1,
**dadurch gekennzeichnet,**
**dass** die Rohstoffe bzw. die aus diesen gebildete Schmelze in dem Behälter bei der Temperatur T_{AU} über die Zeit t_{H} mit 2 h ≤ t_{H} ≤ 7 h gehalten wird.

5. Verfahren nach zumindest Anspruch 1,
**dadurch gekennzeichnet,**
**dass** die Schmelze in dem Behälter durch Thermik homogenisiert wird, wobei ggfs. während des Homogenisierens die Schmelze in dem Behälter abgekühlt wird.

6. Verfahren nach zumindest Anspruch 1,
**dadurch gekennzeichnet,**
**dass** die Schmelze in dem Behälter in dessen Auslassbereich auf die Temperatur T_{AB} abgekühlt wird, insbesondere mit 1200 °C ≤ T_{AB} ≤ 1300 °C.

7. Verfahren nach zumindest Anspruch 1,
**dadurch gekennzeichnet,**
**dass** der Stabilisator mehr als 90 Gew.-% ZrO₂, insbesondere mehr als 95 Gew.% ZrO₂, bevorzugterweise mehr als 97, 5 Gew.-% ZrO₂ enthält.

8. Verfahren nach zumindest Anspruch 1,
**dadurch gekennzeichnet,**
**dass** nach Schmelzen der Rohstoffe in dem Behälter und Homogenisieren in diesem, insbesondere durch Thermik, die Schmelze unmittelbar in die Behältnisse abgefüllt wird.

9. Verfahren nach zumindest einem der vorhergehenden Ansprüche, **dadurch gekennzeichnet,**
**dass** die Zusammensetzung des Rohlings in Gew.-% enthält:
| | |
|---|---|
| SiO2 | 46,0 - 72,0 |
| Li2O | 10,0 - 25,0 |
| ZrO₂ | 8,0 - 20,0 |
| Al2O3 | 0,1 - 8,0 |
| K2O | 0,1 - 5,0 |
| CeO2 | 0,0 - 4,0 |
| B2O3 | 0,0 - 4,0 |
| Na2O | 0,0 - 4,0 |
| Tb4O7 | 0,0 - 2,5 |
| zumindest einen Keimbildner 1,0 - 10,0, wie P2O5, sowie 0,0 bis 4,0 von zumindest einem Additiv, | |
wobei das Additiv zumindest ein Oxid aus der Gruppe BaO, CaO, MgO, MnO, Er₂O₃, Pr₆O₁₁, Sm₂O₃, TiO₂, V₂O_{S}, Y₂O₃ ist,
und die Gesamtsumme 100 Gew.-% ausmacht.

10. Verfahren nach zumindest einem der vorhergehenden Ansprüche,
**dadurch gekennzeichnet,**
**dass** die Zusammensetzung des Rohlings in Gew.-% ist
| | |
|---|---|
| SiO2 | 58 - 60 |
| Li2O | 13,5 - 20,5 |
| ZrO₂ | 9,0 - 12,5 |
| Keimbildner, insbesondere P2O5, | 3,0 - 7,5 |
| Al2O3 | 0,5 - 6,0 |
| K2O | 0,5 - 3,5 |
| CeO2 | 0,5 - 2,5 |
| B2O3 | 0 - 3 |
| Na2O | 0 - 3 |
| Tb4O7 | 0 -1,5 , |
wobei die Gesamtsumme 100 Gew.-% ist.

11. Verfahren nach zumindest Anspruch 1, **dadurch gekennzeichnet,**
**dass** die in den Behältnissen abgefüllte und in diesen abgekühlte Schmelze zumindest einer ersten Wärmebehandlung W1 bei einer Temperatur T_{W1} über einen Zeitraum t_{W1} unterzogen wird, wobei 620 °C ≤ T_{W1} ≤ 800 °C und/oder 1 min ≤ t_{W1} ≤ 200 min, ist.

12. Verfahren nach Anspruch 11,
**dadurch gekennzeichnet,**
**dass** die erste Wärmebehandlung W1 in zwei Stufen durchgeführt wird, wobei insbesondere in der ersten Stufe eine Temperatur T_{St1} mit 630 °C ≤ T_{St1} ≤ 690 °C und/oder in der zweiten Stufe eine Temperatur T_{ST2} mit 720 °C ≤ Tst2 ≤ 780 °C eingestellt wird.

13. Verfahren nach Anspruch 11 oder 12,
**dadurch gekennzeichnet,**
**dass** der Lithiumsilikat-Glaskeramikrohling nach der ersten Wärmebehandlung W1 einer zweiten Wärmebehandlung W2 bei einer Temperatur T_{W2} über eine Zeit t_{W2} unterzogen wird, wobei 800 °C ≤ T_{W2} ≤ 1040 °C, vorzugsweise 800 °C ≤ T_{W2} ≤ 900 °C, und/oder 5 min ≤ t_{W2} ≤ 200 min, vorzugsweise 5 min ≤ t_{W2} ≤ 30 min, ist.

14. Verfahren zur Herstellung eines Dentalprodukts, wobei zunächst ein Lithiumsilikat-Glaskeramikrohling nach einem der Ansprüche 11 bis 13 hergestellt und sodann durch CAD/CAM-Verfahren aus dem Glaskeramikrohling das Dentalprodukt hergestellt wird.

## Claims

1. A method for producing a lithium silicate glass preform having a composition with at least 8% by weight, preferably 9 to 20% by weight, of a stabiliser from the group ZrO₂, HfO₂ or mixtures thereof, comprising the method steps
- mixing raw materials containing the stabiliser in powder form, the powder having a particle size d₅₀ = x with 0.3 µm ≤ x ≤ 1.5 µm,
- melting the raw materials at a temperature T_{AU} with 1450°C ≤ T_{AU} ≤ 1600°C in a vessel and holding the melt in the vessel for a time t_{H} with t_{H} ≥ 1 h,
- filling the homogenised melt into containers, the outflow temperature T_{AB} from the vessel being T_{AU} - Y°C = T_{AB} with 150°C ≤ Y ≤ 350°C, the filling of the containers and shaping of the melt in the same having a cooling rate A with 5 K/sec ≤ A ≤ 100 K/sec up to a temperature T_{M} with 600°C ≤ T_{M}, in particular 600°C ≤ T_{M} ≤ 650°C.

2. The method according to claim 1,
**characterised in that**
the powder used also has a grain size d₁₀ = 0.5 • x and/or d₉₀ = 1.5 • x, in particular d₁₀ = 0.5 • x and d₉₀ = 1.5 • x, with 0.3 µm ≤ x ≤ 1.5 µm.

3. The method according to claim 1 or 2,
**characterised in that**
the melt has a temperature T_{B} with 1150°C ≤ T_{B} < T_{AB} during filling of the containers.

4. The method according to claim 1,
**characterised in that**
the raw materials or the melt formed therefrom is kept in the vessel at the temperature T_{AU} for the time t_{H} with 2 h ≤ t_{H} ≤ 7 h.

5. The method according to at least claim 1,
**characterised in that**
the melt in the vessel is homogenised by thermal action, with the melt in the vessel being cooled if necessary during the homogenisation.

6. The method according to at least claim 1,
**characterised in that**
the melt in the vessel in its outlet region is cooled to the temperature T_{AB}, in particular with 1200°C ≤ T_{AB} ≤ 1300°C.

7. The method according to at least claim 1,
**characterised in that**
the stabiliser contains more than 90% by weight ZrO₂, in particular more than 95% by weight ZrO₂, preferably more than 97.5% by weight ZrO₂.

8. The method according to at least claim 1,
**characterised in that**
the melt is filled directly into the containers, after the raw materials have been melted in the vessel and homogenised therein, in particular by thermal action.

9. The method according to at least one of the preceding claims,
**characterised in that**
the composition of the preform contains the following in % by weight:
| | |
|---|---|
| SiO2 | 46.0 - 72.0 |
| Li2O | 10.0 - 25.0 |
| ZrO₂ | 8.0 - 20.0 |
| Al2O3 | 0.1 - 8.0 |
| K2O | 0.1 - 5.0 |
| CeO2 | 0.0 - 4.0 |
| B2O3 | 0.0 - 4.0 |
| Na2O | 0.0 - 4.0 |
| Tb4O7 | 0.0 - 2.5 |
| at least one nucleating agent 1.0 - 10.0, such as P2O5, and 0.0 to 4.0 of at least one additive, | |
wherein the additive is at least one oxide from the group BaO, CaO, MgO, MnO, Er₂O3, Pr₆O₁₁, Sm₂O₃, TiO₂, V₂O₅, Y₂O₃,
and the total is 100% by weight.

10. The method according to at least one of the preceding claims,
**characterised in that**
the composition of the preform is in % by weight
| | |
|---|---|
| SiO2 | 58 - 60 |
| Li2O | 13.5 - 20.5 |
| ZrO₂ | 9.0 - 12.5 |
| nucleating agent, in particular P2O5, | 3.0 - 7.5 |
| Al2O3 | 0.5-6.0 |
| K2O | 0.5 - 3.5 |
| CeO2 | 0.5 - 2.5 |
| B2O3 | 0 - 3 |
| Na2O | 0 - 3 |
| Tb4O7 | 0 - 1.5, |
the total being 100% by weight.

11. The method according to at least claim 1,
**characterised in that**
the melt filled into the containers and cooled therein is subjected to at least a first heat treatment W1 at a temperature T_{W1} over a period of time t_{w1}, where 620°C ≤ T_{W1}, ≤ 800°C and/or 1 min ≤ t_{w1} ≤ 200 min.

12. The method according to claim 11,
**characterised in that**
the first heat treatment W1 is carried out in two stages, wherein, in particular in the first stage, a temperature T_{St1} is set with 630°C ≤ T_{St1} ≤ 690°C and/or in the second stage a temperature T_{ST2} is set with 720°C ≤ T_{St2} ≤ 780°C.

13. The method according to claim 11 or 12,
**characterised in that**
the lithium silicate glass ceramic preform after the first heat treatment W1 is subjected to a second heat treatment W2 at a temperature T_{W2} for a time t_{W2}, wherein 800°C ≤ T_{W2} ≤ 1040°C, preferably 800°C ≤ T_{W2} ≤ 900°C, and/or 5 min ≤ t_{W2} ≤ 200 min, preferably 5 min ≤ t_{W2} ≤ 30 min.

14. A method for producing a dental product, in which first a lithium silicate glass ceramic preform according to one of claims 11 to 13 is produced and then the dental product is produced from the glass ceramic preform by CAD/CAM methods.

## Revendications

1. Procédé de fabrication d'une ébauche en verre de silicate de lithium ayant une composition contenant au moins 8 % en poids, de préférence 9 à 20 % en poids, d'un stabilisant du groupe ZrO₂, HfO₂ ou leurs mélanges, comprenant les étapes de procédé consistant à
- mélanger les matières premières contenant le stabilisant sous forme de poudre, la poudre ayant une granulométrie d₅₀ = x, avec 0,3 µm ≤ x ≤ 1,5 µm,
- faire fondre les matières premières à une température T_{AU}, 1450 °C ≤ T_{AU} ≤ 1600 °C, dans un récipient et maintenir la masse fondue dans le récipient pendant un temps t_{H}, avec t_{H} ≥ 1 h,
- verser la masse fondue homogénéisée dans des contenants, la température d'écoulement T_{AB} du récipient s'élevant à T_{AU} - Y °C = T_{AB}, avec 150 °C ≤ Y ≤ 350 °C, le remplissage des contenants et la mise en forme de la masse fondue dans ceux-ci s'effectuant à une vitesse de refroidissement A avec 5 K/s ≤ A ≤ 100 K/s jusqu'à une température T_{M} avec 600 °C ≤ T_{M}, notamment 600 °C ≤ T_{M} ≤ 650 °C.

2. Procédé selon la revendication 1,
**caractérisé en ce que**
la poudre utilisée a également une granulométrie d₁₀ = 0,5 • x et/ou d₉₀ = 1,5 • x, en particulier d₁₀ = 0,5 • x et d₉₀ = 1,5 • x, avec 0,3 µm ≤ x ≤ 1,5 µm.

3. Procédé selon la revendication 1 ou la revendication 2,
**caractérisé en ce que**
la masse fondue a une température T_{B}, avec 1150 °C ≤ T_{B} < T_{AB}, lors du remplissage des contenants.

4. Procédé selon la revendication 1,
**caractérisé en ce que**
les matières premières ou la masse fondue formée à partir de celles-ci sont maintenues dans le récipient à la température T_{AU} pendant le temps t_{H}, avec 2 h ≤ t_{H} ≤ 7 h.

5. Procédé selon au moins la revendication 1,
**caractérisé en ce que**
la masse fondue dans le récipient est homogénéisée par action thermique, la masse fondue étant refroidie dans le récipient, si nécessaire, pendant l'homogénéisation.

6. Procédé selon au moins la revendication 1,
**caractérisé en ce que**
la masse fondue dans le récipient est refroidie dans sa zone de sortie à la température T_{AB}, en particulier 1200 °C ≤ T_{AB} ≤ 1300 °C.

7. Procédé selon au moins la revendication 1,
**caractérisé en ce que**
le stabilisant contient plus de 90 % en poids de ZrO₂, en particulier plus de 95 % en poids de ZrO₂, de préférence plus de 97,5 % en poids de ZrO₂.

8. Procédé selon au moins la revendication 1,
**caractérisé en ce que**
une fois les matières premières fondues dans le récipient et homogénéisées dans celuici, notamment par action thermique, la masse fondue est versée directement dans les contenants.

9. Procédé selon au moins une des revendications précédentes,
**caractérisé en ce que**
la composition de l'ébauche contient en % en poids :
| | |
|---|---|
| SiO2 | 46,0 - 72,0 |
| Li2O | 10,0 - 25,0 |
| ZrO₂ | 8,0 - 20,0 |
| Al2O3 | 0,1 - 8,0 |
| K2O | 0,1 - 5,0 |
| CeO2 | 0,0 - 4,0 |
| B2O3 | 0,0 - 4,0 |
| Na2O | 0,0 - 4,0 |
| Tb4O7 | 0,0 - 2,5 |
| au moins un agent de nucléation 1,0 - 10,0, tel que P2O5, et 0,0 - 4,0 d'au moins un additif, | |
l'additif étant au moins un oxyde du groupe BaO, CaO, MgO, MnO, Er₂O3, Pr₆O₁₁, Sm₂O₃, TiO₂, V₂O₅, Y₂O₃,
pour faire au total, 100 % en poids.

10. Procédé selon au moins une des revendications précédentes,
**caractérisé en ce que**
la composition de l'ébauche est constituée de, en % en poids
| | |
|---|---|
| SiO2 | 58 - 60 |
| Li2O | 13,5 - 20,5 |
| ZrO₂ | 9,0 - 12,5 |
| agent de nucléation, en particulier P2O5, | 3,0 - 7,5 |
| Al2O3 | 0,5 - 6,0 |
| K2O | 0,5 - 3,5 |
| CeO2 | 0,5 - 2,5 |
| B2O3 | 0 - 3 |
| Na2O | 0 - 3 |
| Tb4O7 | 0 - 1,5, |
pour faire au total, 100 % en poids.

11. Procédé selon au moins la revendication 1,
**caractérisé en ce que**
la masse fondue versée dans les contenants et refroidie dans ceux-ci est soumise à au moins un premier traitement thermique W1 à une température T_{W1} pendant une période de temps t_{w1}, où 620 °C ≤ T_{W1} ≤ 800 °C et/ou 1 min ≤ t_{w1} ≤ 200 min.

12. Procédé selon la revendication 11,
**caractérisé en ce que**
le premier traitement thermique W1 est effectué en deux étapes, où en particulier, dans la première étape, une température T_{St1} est réglée à 630 °C ≤ T_{St1} ≤ 690 °C et/ou dans la seconde étape, une température T_{ST2} est réglée à 720 °C ≤ T_{St2} ≤ 780 °C.

13. Procédé selon la revendication 11 ou 12,
**caractérisé en ce que**
l'ébauche en vitrocéramique de silicate de lithium après le premier traitement thermique W1 est soumise à un second traitement thermique W2 à une température T_{W2} pendant un temps t_{W2}, où 800 °C ≤ T_{W2} ≤ 1040 °C, de préférence 800 °C ≤ T_{W2} ≤ 900 °C, et/ou 5 min ≤ t_{W2} ≤ 200 min, de préférence 5 min ≤ t_{W2} ≤ 30 min.

14. Procédé de fabrication d'un produit dentaire, dans lequel une ébauche en vitrocéramique au silicate de lithium selon l'une des revendications 11 à 13 est d'abord produite, puis le produit dentaire est fabriqué à partir de l'ébauche en vitrocéramique selon des procédés CAO/FAO.
